(19) Europäisches Patentamt
European Patent Office
Office européen des brevets

(11) **EP 2 174 580 A1**

(12) **EUROPEAN PATENT APPLICATION**
published in accordance with Art. 153(4) EPC

(43) Date of publication:
**14.04.2010 Bulletin 2010/15**

(51) Int Cl.:
***A61B 1/00*** *(2006.01)*          ***A61B 5/07*** *(2006.01)*

(21) Application number: **08765113.9**

(86) International application number:
**PCT/JP2008/060300**

(22) Date of filing: **04.06.2008**

(87) International publication number:
**WO 2009/022490 (19.02.2009 Gazette 2009/08)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **10.08.2007 JP 2007210378**

(71) Applicant: **Olympus Corporation
Tokyo 151-0072 (JP)**

(72) Inventor: **MATSUDA, Takehiro
Tokyo 151-0072 (JP)**

(74) Representative: **Schmidt, Steffen J.
Wuesthoff & Wuesthoff
Patent- und Rechtsanwälte
Schweigerstrasse 2
81541 München (DE)**

(54) **IMAGE PROCESSING DEVICE, IMAGE PROCESSING PROGRAM, AND IMAGE PROCESSING METHOD**

(57)     A display control unit (120) includes an image display control unit (121) and a display-time-information display control unit (122). The image display control unit (121) sequentially displays a series of intracelomic images on an image display unit (141) based on image information of a series of intracelomic images stored in an image information storage unit (131) and display time information stored in a display-time-information storage unit (132). The display-time-information display control unit (122) displays on a display-time-information display unit (142), display-time information of an image which is displayed posterior in time series at least to an image displayed on the image display unit (141).

FIG.2

**Description**

TECHNICAL FIELD

The present invention relates to an image processing apparatus, an image processing program, and an image processing method for sequentially displaying a plurality of images, in particular, intracelomic images picked up (or taken) in a gastroenteric tract.

BACKGROUND ART

In recent years, a swallowable capsule endoscope which is swallowed by a patient, i.e., a subject from the mouth, and introduced inside the subject is proposed in a field of endoscope. The capsule endoscope picks up several tens of thousands of intracelomic images in, for example, an esophagus, a stomach, a small intestine, and a large intestine, after being swallowed from the mouth of the patient until naturally excreted. A doctor, a nurse, or others who make observation and diagnosis (referred to below as "examiner") needs to observe the enormous amount of intracelomic images.

Conventionally, image processing apparatuses have been developed to reduce the burden of the examiner. In one conventional image processing apparatus, a motion vector between images is calculated and a degree of similarity between the images is determined based on the motion vector. Then, a display rate of each image is set based on the degree of similarity between the images. Thus, view time of intracelomic images is shortened (see Patent Document 1).

Patent Document 1: Japanese Patent Application Laid-Open No. 2006-280792
Patent Document 2: Japanese Patent Application Laid-Open No. 2005-192880
Nonpatent Literature 1: *Digital Image Processing,* Computer Graphic Arts Society (CG-ARTS Society), p202, July 22, 2004

DISCLOSURE OF INVENTION

PROBLEM TO BE SOLVED BY THE INVENTION

The capsule endoscope picks up an image moving with peristalsis. The examiner cannot predict where the contents of images drastically change and where the contents of images do not change substantially, in a series of intracelomic images, and therefore the examiner cannot predict the degree of similarity between images. Hence, when using a conventional image processing apparatus, the examiner has to observe the series of intracelomic images as the display time and the like of each image changes, even though the examiner cannot predict the display time and display rate of each image.

The present invention is made in view of the foregoing, and an object of the present invention is to provide an image processing apparatus, image processing program, and image processing method that can grasp a display time and the like of each image before each image is displayed when a series of images are sequentially displayed according to a display time or a display rate set for each image.

MEANS FOR SOLVING PROBLEM

To solve the problems as described above, and to achieve an object, an image processing apparatus according to the present invention includes an image storage unit that stores a series of images, a display-time-information storage unit that stores display-time information which defines display time of each image, an image display unit that sequentially displays each image based on the display-time information, a display-time-information display unit that displays the display-time information at least of images to be displayed in time series after an image currently displayed on the image display unit, and a display control unit that performs a control to display an image on the image display unit, and to display the display-time information on the display-time-information display unit, based on the images stored in the image storage unit and the display-time information stored in the display-time-information storage unit.

Further, an image processing program according to the present invention causes a computer to perform a storing process to store display-time information which defines display time of each image stored in an image storage unit that stores a series of images, and a display control process to perform a control to sequentially display each image stored in the image storage unit on an image display unit according to the display-time information, and to display the display-time information at least of images to be displayed in time series after an image currently displayed on the image display unit based on the display-time information stored in the display-time-information storage unit.

Still further, an image processing method according to the present invention includes a storing step of storing display-time information which defines display time of each image stored in an image storage unit that stores a series of images, and a display control step of performing a control to sequentially display each image stored in the image storage unit on an image display unit according to the display-time information, and to display the display-time information at least of images to be displayed in time series after an image currently displayed on the image display unit based on the display-time information stored in the display-time-information storage unit.

EFFECT OF THE INVENTION

The image processing apparatus according to the present invention has an advantageous effect in that, the display control unit sequentially displays a series of images on the image display unit according to the display-time information, and displays on the display-time-information display unit the display-time information of at least images to be displayed in time series after an image currently displayed on the image display unit. Therefore, the examiner can grasp the display time and the like of each image before each image is displayed so as to observe each image following the change of display time. The oversight of the images decreases and the burden of reviewing the images can be lightened.

BRIEF DESCRIPTION OF DRAWINGS

FIG. 1 is a diagram of a schematic configuration of an intra-subject information acquiring system according to a first embodiment of the present invention.
FIG. 2 is a schematic block diagram of a configuration of an image processing apparatus according to the first embodiment of the present invention.
FIG. 3 is a view of an example of a display screen of a display unit shown in FIG. 2.
FIG. 4 is a flowchart of a procedure of play processes of a series of intracelomic images, performed by the image processing apparatus shown in FIG. 2.
FIG. 5 is a view of an example of a display screen of the display unit shown in FIG. 2.
FIG. 6 is a schematic block diagram of a configuration of an image processing apparatus according to a second embodiment of the present invention.
FIG. 7 is a view of an example of a display screen of a display unit shown in FIG. 6.
FIG. 8 is a flowchart of a procedure of play processes of a series of intracelomic images, performed by the image processing apparatus shown in FIG. 6.
FIG. 9 is a schematic block diagram of a configuration of an image processing apparatus according to a third embodiment of the present invention.
FIG. 10 is a view of an example of a display screen of a display unit shown in FIG. 9.

EXPLANATIONS OF LETTERS OR NUMERALS

| | |
|---|---|
| 1 | Subject |
| 2 | Capsule endoscope |
| 3 | Receiving apparatus |
| 4 | Recording medium |
| 5, 6, 7 | Image processing apparatus |
| 10, 20, 30 | Control unit |
| 11 | Input unit |
| 12 | Card I/F |
| 13, 23, 33 | Storage unit |
| 14, 24, 34 | Display unit |
| 110, 210, 310 | Image processing unit |
| 111 | Display-time-information calculating unit |
| 120, 220, 320 | Display control unit |
| 121, 321 | Image display control unit |
| 122, 322 | Display-time-information display control unit |
| 123 | Display scope switch unit |

| 131 | Image information storage unit |
| 132 | Display-time-information storage unit |
| 141 | Image display unit |
| 142, 342 | Display-time-information display unit |
| 212 | Average color calculating unit |
| 213 | Lesion information calculating unit |
| 224 | Color bar display control unit |
| 225 | Lesion bar display control unit |
| 233 | Average color storage unit |
| 234 | Lesion information storage unit |
| 243 | Color bar display unit |
| 244 | Lesion bar display unit |
| 312 | Display-time-information changing unit |
| 333 | Change storage unit |
| W1 | Diagnosis window |
| W2, W3, W4, W5 | Information display window |
| F1 | Image display area |
| F2 | Image ID information display area |
| F3 | Forward button |
| F4 | Backward button |
| F5, F8, F11, F14 | Display-time-graph display area |
| F6, F9 | Play position |
| F7, F10 | Display scope switch button |
| F12 | Color bar display area |
| F13 | Lesion bar display area |
| F15, F16 | Information change button |
| F17 | Change save button |

BEST MODE(S) FOR CARRYING OUT THE INVENTION

Exemplary embodiments of an image processing apparatus, an image processing program, and an image processing method according to the present invention are described below in detail with reference to accompanying drawings. The present invention, however, is not limited to the embodiments.

First Embodiment

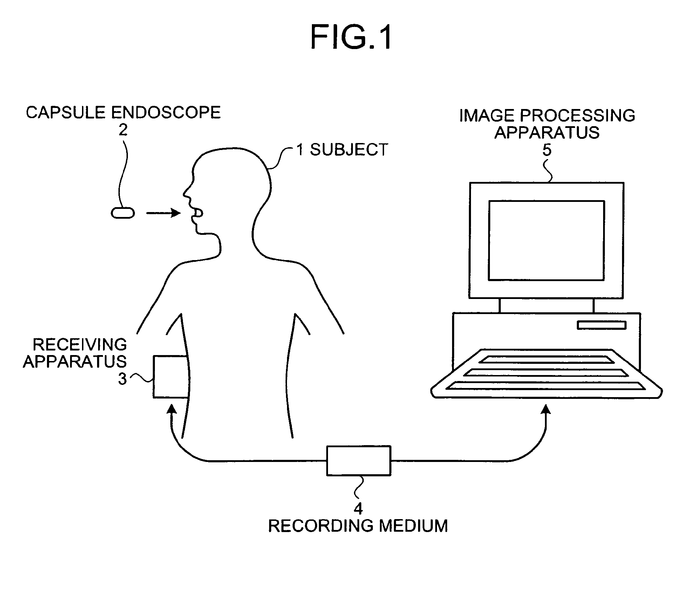

FIG. 1 is a schematic diagram of an example of a configuration of an intra-subject information acquiring system that includes an image processing apparatus according to a first embodiment of the present invention. As shown in FIG. 1, the intra-subject information acquiring system is configured with a capsule endoscope 2 that picks up intracelomic images inside a subject 1, a receiving apparatus 3 that receives image information of intracelomic images radio transmitted from the capsule endoscope 2, an image processing apparatus 5 that processes the intracelomic images picked up by the capsule endoscope 2 based on the image information received by the receiving apparatus 3, and the like. A recording medium 4 is used for transfer of the image information between the receiving apparatus 3 and the image processing apparatus 5.

The capsule endoscope 2, which is introduced inside the subject 1, has an imaging function to sequentially pick up images inside the subject 1 in time series, and a radio communication function to transmit radio signals including the picked-up images to an outside. The capsule endoscope 2 is swallowed by the subject 1, travels inside a body cavity with peristalsis of a gastroenteric tract, sequentially picks up images inside the subject 1 at predetermined intervals, for example, of 0.5 second, and sequentially transmits the images inside the subject 1 to the receiving apparatus 3 via predetermined electric waves as needed.

The receiving apparatus 3 stores in the recording medium 4 information such as received images and imaging time of each image as image information as needed. The recording medium 4 is realized with a portable recording medium such as a CompactFlash®. The recording medium 4 is attachable/detachable to/from the receiving apparatus 3 and the image processing apparatus 5, and has such a configuration that the information can be output therefrom and recorded therein when attached to the receiving apparatus 3 and the image processing apparatus 5.

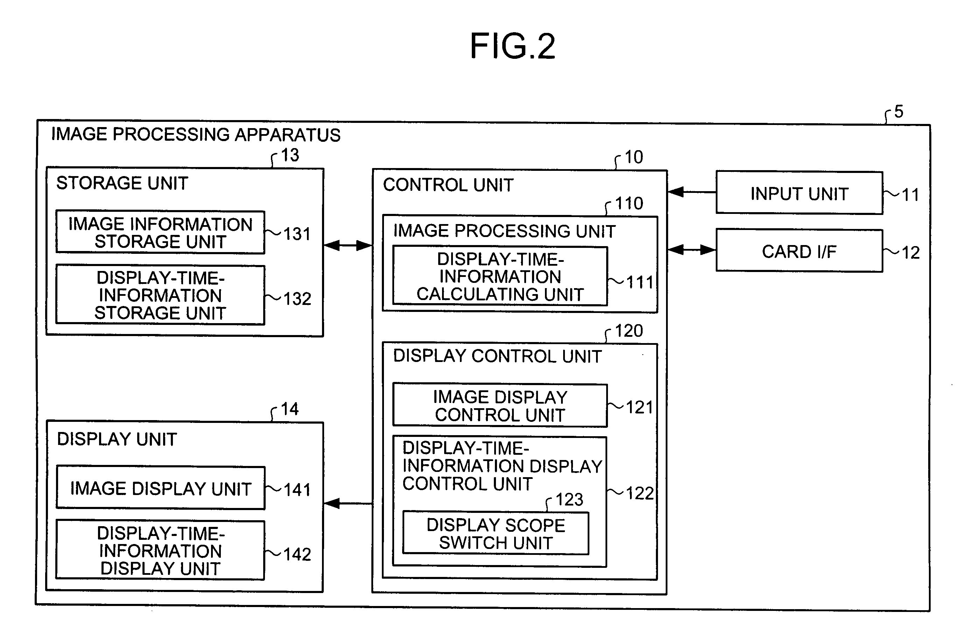

The image processing apparatus 5 has an image display function to take in the image information stored in the recording medium 4 by the receiving apparatus 3, and to sequentially display (referred to below as "play") intracelomic images of the subject 1 in an order of imaging. The examiner makes the image processing apparatus 5 play the intracelomic images, and observes (i.e. examines) the inside of the subject 1 such as an esophagus, a stomach, a small intestine, and a large intestine. The image processing apparatus 5 is configured in a similar manner as a workstation. To be specific, the image processing apparatus 5 has a control unit 10, an input unit 11, a card interface (I/F) 12, a storage unit 13, and a display unit 14, as shown in FIG. 2.

The control unit 10 controls various kinds of processes performed by each unit of the image processing apparatus 5, and controls input/output of information among the units. The control unit 10 includes, in particular, an image processing unit 110 that processes image information, and a display control unit 120 that controls display processes in the display unit 14.

The image processing unit 110 includes, in particular, a display-time-information calculating unit 111 that processes the image information and calculates display-time information for determining display time of each image. The display control unit 120 includes an image display control unit 121 that controls the display unit 14 to play each image, and a display-time-information display control unit 122 that controls display of the display-time information of each image, based on the image information and the display-time information.

The input unit 11 is realized with a keyboard, a mouse, and the like and inputs various kinds of information to the control unit 10 according to an input manipulation by the examiner.

The card I/F 12, to which the recording medium 4 is removably attached, reads out the image information and image ID information stored in the recording medium 4, and transfers the read-out information to the control unit 10. Further, the card I/F 12 writes into the recording medium 4 information for which the control unit 10 gives a write instruction, for example, the image ID information. The image ID information includes, for example, a name, a sex, and a birth date of the subject 1, and an image ID.

The storage unit 13 is realized with an information recording medium in which information can be stored and from which information can be read out, such as a random access memory (RAM), an electrically erasable programmable read-only memory (EEPROM), and a hard disk. The storage unit 13 stores information for which the control unit 10 gives a write instruction, and supplies information for which the control unit 10 gives a read instruction to the control unit 10. The storage unit 13 includes an image information storage unit 131 and a display-time-information storage unit 132. The image information storage unit 131 stores the image ID information corresponding to a series of intracelomic images and the image information of the series of intracelomic images. The display-time-information storage unit 132 stores the display-time information of each intracelomic image stored in the image information storage unit 131.

The display unit 14 is realized with various kinds of displays such as a CRT display and a liquid crystal display, and displays various kinds of information for which the display control unit 120 gives a display instruction. In particular, the display unit 14 includes an image display unit 141 that displays the intracelomic image, and a display-time-information display unit 142 that displays the display-time information. Thus, the display unit 14 displays various kinds of information necessary for observation and diagnosis of the inside of the body cavity of the subject 1.

The CPU in the image processing apparatus 5 which includes the units as described above reads out from the storage unit 13 an image processing program for realizing processes performed by the image processing apparatus 5 of the embodiment and executes the same. The image processing program can be recorded in a computer-readable recording medium such as a flexible disc, a CD-ROM, a DVD-ROM, and a flash memory so as to be widely distributed. Therefore, the image processing apparatus 5 according to the embodiment may include an auxiliary storage device which can read out information from at least one of various types of recording medium as listed above.

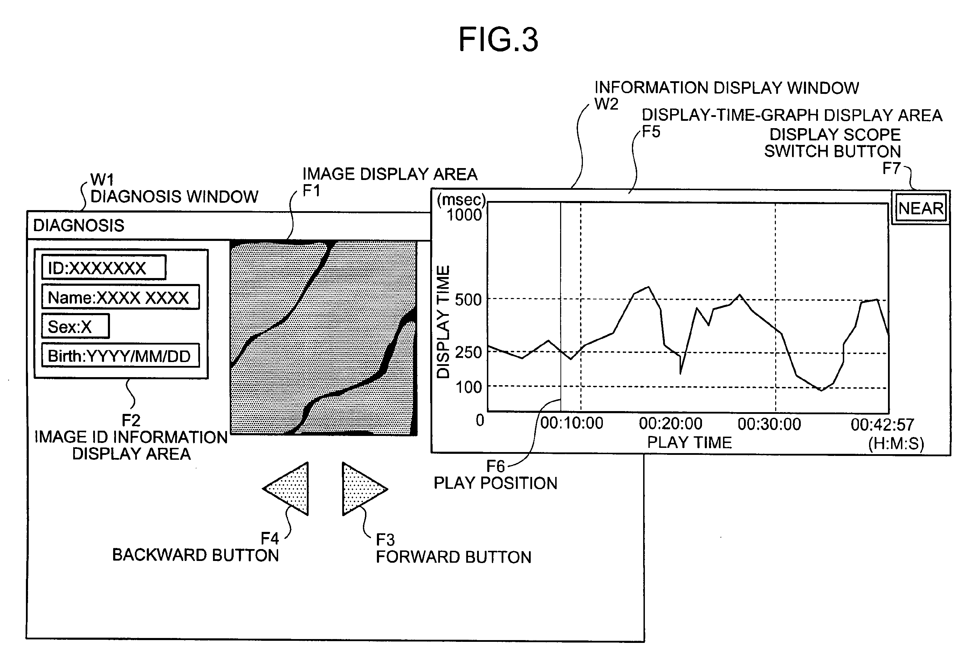

Now, a specific example of a display screen of the display unit 14 is described below with reference to FIG. 3. FIG. 3 is a view of a diagnosis window W1 and an information display window W2, which are examples of the display screen. The diagnosis window W1 includes an image display area F1 in which the intracelomic images are played, an image ID information display area F2 in which the image ID information is displayed, a forward button F3, and a backward button F4. The information display window W2 includes a display-time-graph display area F5 and a display scope switch button F7. Here, the image display area F1 represents a specific mode of the image display unit 141, and the display-time-graph display area F5 represents a specific mode of the display-time-information display unit 142.

In the image display area F1, under the control of the image display control unit 121, each image in the series of intracelomic images, which is a play target, is played, based on the display-time information that is calculated beforehand. In the display-time-graph display area F5, under the control of the display-time-information display control unit 122, a graph showing a relation between information specifying each image in time series in the series of intracelomic images and the display-time information of each image (the graph is referred to below as "display time graph") is displayed. For example, the display time graph shows a relation between play time and display time, plotting the play time, which is time spent until each image is displayed after the playing of the intracelomic images starts, along a horizontal axis, and plotting the display time of each image along a vertical axis, as shown in FIG. 3.

Here, the information specifying each image in time series is not limited to the play time, and may be imaging time indicating the time each image is picked up, or an image number which is a number assigned to each image in the order of imaging. Further, the display-time information is not limited to the display time, and may be a frame rate that indicates the number of images (i.e. frames) displayed per unit time, a value that determines a relative display time of each image, or a coefficient that determines the display time or the frame rate of each image.

Further, in the display-time-graph display area F5, under the control of the display-time-information display control unit 122, a play position F6, which is a marker indicating a position of an image currently displayed in the image display area F1 in time series in the series of intracelomic images, is displayed. Specifically, the play position F6 is a line parallel to a display time axis which is the vertical axis, moves along a play time axis which is the horizontal axis, and indicates play time corresponding to the image currently displayed in the image display area F1. When the examiner forcibly moves the play position F6 on the display time graph, for example, by dragging the play position F6 using a mouse of the input unit 11, the image display control unit 121 displays an intracelomic image corresponding to play time indicated by the moved play position F6 in the image display area F1. Alternatively, the display time graph may be shown in different colors in a right-side area and a left-side area of a position corresponding to the play time of the image displayed in the image display area F1, for example, so as to indicate the play position.

The diagnosis window W1 is a GUI (Graphical User Interface) screen. The examiner selects the forward button F3 or the backward button F4, for example, by clicking a mouse of the input unit 11 to input a play instruction on images into the display control unit 120. While each image is played, the selected button changes into a play stop button not shown. The examiner inputs a play stop instruction into the display control unit 120 by selecting the play stop button. When the forward button F3 is selected, the image display control unit 121 plays the series of intracelomic images in an order of time series according to the time the images are picked up, whereas, when the backward button F4 is selected, the image display control unit 121 plays the series of intracelomic images in a reverse order of time series according to the time the images are picked up.

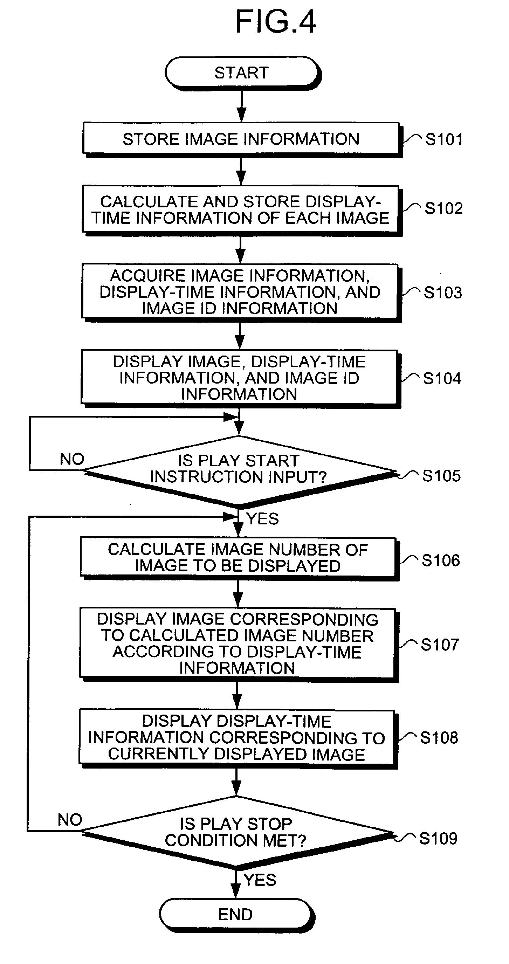

Next, a procedure to play the series of intracelomic images on the display unit 14, performed by the control unit 10 is described below with reference to FIG. 4. Firstly, the image processing unit 110 acquires the image information of the series of intracelomic images from the recording medium 4 via the card I/F 12, and stores the image information in the image information storage unit 131 (Step S101). The display-time-information calculating unit 111 processes the image information and calculates the display-time information of each image, and stores the display-time information in the display-time-information storage unit 132 (Step S102). Then, the display control unit 120 acquires information needed for the examiner to diagnose the subject 1, such as the image information, the display-time information, and the image ID information (Step S103). Next, respective units of the display control unit 120 displays the image, the display-time information, the image ID information, and the like on the display unit 14 (Step S104). Here, the image display control unit 121 displays, for example, an image picked up first in the series of intracelomic images in the image display area F1. At this point, the examiner can grasp time required for playing the series of intracelomic images, that is, total play time, and how the display time of images changes over the play time.

Next, the display control unit 120 determines whether a play start instruction is input (Step S105). If the play start instruction is input (Step S105: Yes), the image display control unit 121 calculates the image number of an image that is to be displayed after the image currently displayed on the image display unit 141 (Step S106). Then, the image display control unit 121 displays an image corresponding to the calculated image number on the image display unit 141 based on the display-time information of the image (Step S107). The display-time-information display control unit 122 displays the display-time information corresponding to the image currently displayed on the image display unit 141 on the display-time-information display unit 142 (Step S108). Specifically, the display-time-information display control unit 122 moves the play position F6 to a position corresponding to the image currently displayed in the image display area F1, that is, to a position corresponding to current play time. While the play instruction on images is on (Step S109: No), the display control unit 120 repeats the processes at Steps S106 to S108. On the other hand, if a play stop condition is met, for example, when a play stop instruction on images is received, or when the play reaches a forward end of the play time-series (Step S109: Yes), the display control unit 120 stops a process of playing the images. As described above, the image processing apparatus 5 plays the series of intracelomic images picked up by the capsule endoscope 2, and at the same time, displays the display-time information of each played image and how the display time of images changes over time.

At Step S102, the display-time-information calculating unit 111 calculates the display-time information of each image based on the image information. In the first embodiment, a case where the display-time-information calculating unit 111 calculates the display time as the display-time information is described. Firstly, the display-time-information calculating unit 111 calculates a degree of similarity (i.e., image similarity) between two images picked up successively using normalized cross-correlation described, for example, in Nonpatent Literature 1. The calculated image similarity takes a value within the range from -1 to 1. When two images are identical, the image similarity takes the maximum value, 1. A manner of calculation of the image similarity is not limited to the manner using normalized cross-correlation, and for example, a manner described in Patent Document 1, which uses a motion vector, may be used.

The display-time-information calculating unit 111 calculates the display time of each image in the series of intracelomic images, which is the play target, using the image similarity and following formula (1). Here in the formula (1), i represents the image number, $T_i$ represents the play time of an image of the image number i, $R_i$ represents image similarity of the image of the image number i, maxTime represents a predetermined maximum value of the display time, and minTime represents a predetermined minimum value of the display time.
[Formula 1]

$$T_i = (\max \text{Time} - \min \text{Time}) \times \left( \frac{1}{2} - \frac{R_i}{2} \right) + \min \text{Time} \quad (1)$$

Information the image processing unit 110 stores in the display-time-information storage unit 132 is not limited to the display-time information calculated by the display-time-information calculating unit 111, and may be display-time information calculated by another examiner or another image processing apparatus.

The display-time-information display control unit 122 controls the display unit 14 to display the display-time information of the entire series of intracelomic images. Further, the display-time-information display control unit 122 controls the display unit 14 to display only the display-time information of near images, in other words, images displayed within a predetermined time period before and after the image currently displayed in the image display area F1. The display-time-information display control unit 122 includes a display scope switch unit 123 that controls the display unit 14 to switch the display of the display-time information.

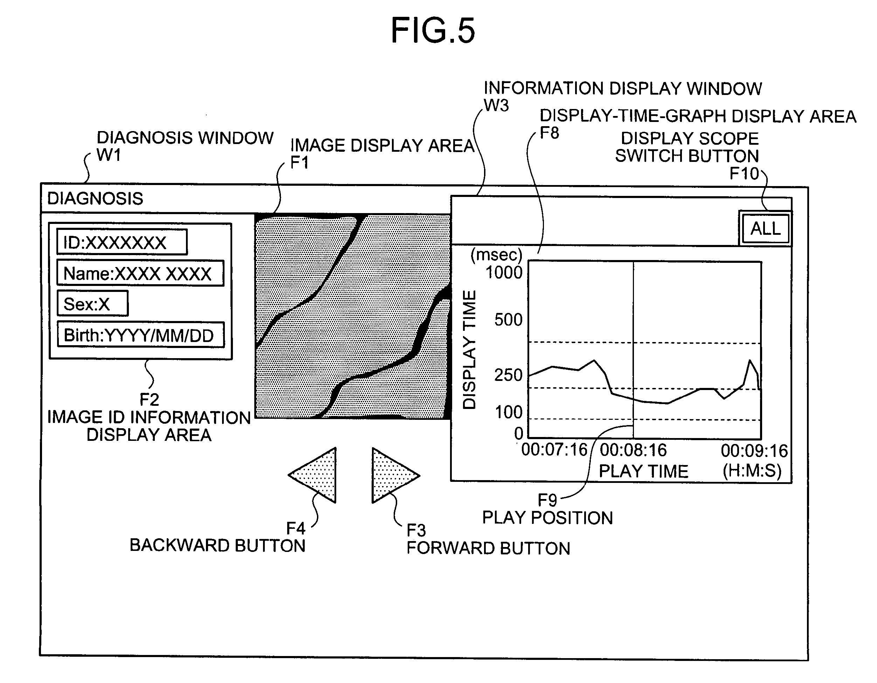

FIG. 5 is a view of a specific example of the display screen where the display-time information of the near images is displayed. As shown in FIG. 5, in an information display window W3, a display-time-graph display area F8 is displayed in place of the display-time-graph display area F5. In the display-time-graph display area F8, a play position F9 is fixedly set in the center of a horizontal axis, i.e., play time axis, and the displayed display time graph corresponds to a time period covering a predetermined time before the play position F9 up to a predetermined time after the play position F9. Alternatively, in the display-time-graph display area F8, the display time of a predetermined number of images before and after the image displayed in the image display area F1 may be displayed.

When the display time graph of the near images alone is displayed, the display-time-information display control unit 122 updates a scale of the play time axis to make it correspond to the image displayed in the image display area F1 at the process of Step S108. Further, the display-time-information display control unit 122 moves the display time graph according to the update of the play time axis. Therefore, in the display-time-graph display area F8, the display time of the images within a predetermined scope along the time series from the image displayed in the image display area F1 is always displayed.

As shown in FIG. 3, the display scope switch button F7 is displayed in the information display window W2. When the examiner selects the display scope switch button F7, the display scope switch unit 123 controls the display unit 14 to display the information display window W3 in place of the information display window W2. Then, an area where the display time graph is shown shrinks, whereby the display screen can be used effectively. For example, the intracelomic image can be displayed in a larger size because the display area of the image display area F1 is not restricted. Further, in this case, since only a small amount of display-time information is displayed, the display-time information of the currently-displayed image and its near images can be displayed in detail.

On the other hand, as shown in FIG. 5, a display scope switch button F10 is displayed in the information display window W3. When the display scope switch button F10 is selected by the examiner, the display scope switch unit 123 controls the display unit 14 to display the information display window W2 in place of the information display window W3. The examiner can select one type of the display time graph depending on a situation. For example, the examiner can refer to the information display window W2 to check how the display time changes over a wider scope, and can refer to the information display window W3 to check the display time of each image in detail.

With the image processing apparatus 5, since the examiner can check the display-time information before each image is displayed, the examiner can observe each image knowing the display time of each image. Further, with the image processing apparatus 5, since the examiner knows when the display time drastically changes, the examiner is less likely to overlook an image, whereby work burden caused by overlooking and reviewing the images can be reduced.

In the description of the first embodiment above, the display-time information of images to be displayed in time series before and after the image currently displayed in the image display area F1 is displayed on the display-time-information display unit 142. The manner the display-time information is displayed is not limited thereby. The display-time-information display unit 142 only needs to display at least the display-time information of images to be displayed in time series after the image currently displayed on the image display unit 141. When the examiner knows at least the display time of images to be displayed later, the examiner can observe each image adjusting his manner of observation to the display time.

Second Embodiment

Next, a second embodiment of the present invention is described below. In the first embodiment, only the display-time information is displayed while the images are played. In the second embodiment, information on an average color of each image and information on a lesion area appearing in each image are further displayed.

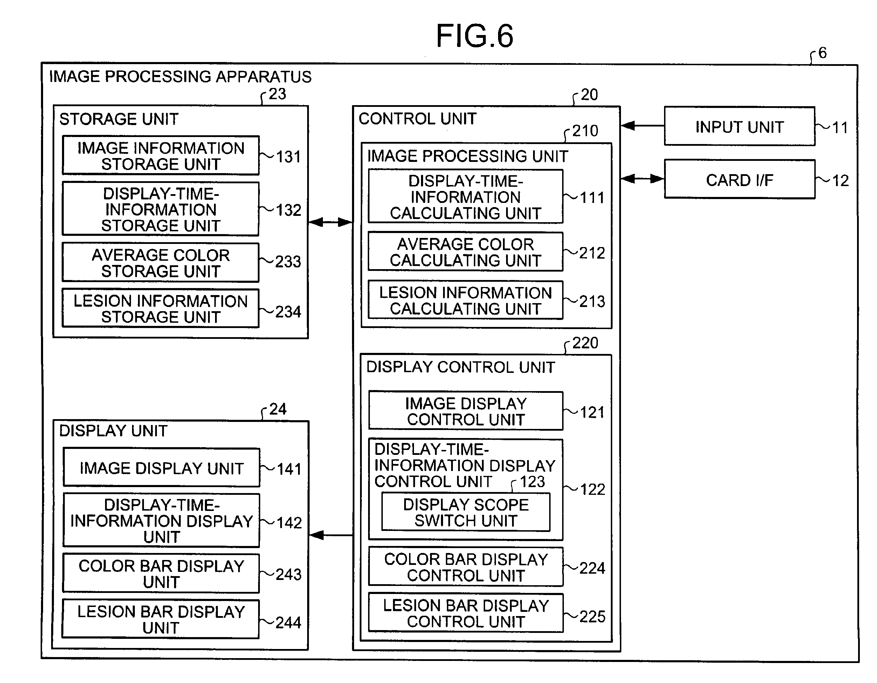

In the second embodiment, an image processing apparatus 6 (not shown) is provided in place of the image processing apparatus 5 in the intra-subject information acquiring system of the first embodiment. FIG. 6 is a schematic block diagram of the image processing apparatus 6. As shown in FIG. 6, the image processing apparatus 6 includes a control unit 20, a storage unit 23, and a display unit 24 in place of the control unit 10, the storage unit 13, and the display unit 14 of the image processing apparatus 5. The control unit 20 includes an image processing unit 210 and a display control unit 220. The image processing unit 210 includes, in addition to the display-time-information calculating unit 111, an average color calculating unit 212 and a lesion information calculating unit 213, and the display control unit 220 includes, in addition to the units included in the display control unit 110, a color bar display control unit 224 and a lesion bar display control unit 225. The storage unit 23 includes, in addition to the units included in the storage unit 13, an average color storage unit 233 and a lesion information storage unit 234. The display unit 24 includes, in addition to the units included in the display unit 14, a color bar display unit 243 and a lesion bar display unit 244. Other configurations are same as the configurations of the intra-subject information acquiring system and the image processing apparatus 5 according to the first embodiment, and a same numeral is attached to a same element.

The average color calculating unit 212 processes the image information stored in the image information storage unit 131, calculates an average color of each image, and stores information of the average color in the average color storage unit 233. The lesion information calculating unit 213 determines whether a lesion area appears in each image or not, and attaches a predetermined label for each kind of the lesion to an image in which a lesion area appears. Further, the lesion information calculating unit 213 attaches a predetermined label, which is different from the label attached to the image in which the lesion area appears, to an image in which no lesion area appears. The lesion information calculating unit 213 stores the information above in the lesion information storage unit 234 as lesion information. Specifically, the lesion information calculating unit 213 maps each pixel or an averaged pixel in the image into a feature space based on the color information as disclosed in Patent Document 2, for example. The lesion information calculating unit 213 identifies a normal cluster and an abnormal cluster after a clustering process, detects a pixel area that belongs to the abnormal cluster as a lesion area, and attaches a predetermined label to each image.

The color bar display control unit 224 creates a color bar based on the information on the average color of each image stored in the average color storage unit 233, and controls the display unit 24 to display the color bar on the color bar display unit 243. The color bar is a strip-shaped display area where an average color of each image in the series of intracelomic images is sequentially arranged at a position corresponding to each image. Since a mucous surface of each organ in a gastroenteric tract has a different color, the examiner can specify an organ that appears in each image by checking a transition of average color on the color bar display unit 243.

The lesion bar display control unit 225 creates a lesion bar based on the lesion information stored in the lesion information storage unit 234, and controls the display unit 24 to display the lesion bar on the lesion bar display unit 244. The lesion bar is a strip-shaped display area where a color corresponding to a label attached to each image by the lesion information calculating unit 213 is sequentially arranged at a position corresponding to each image. The examiner can specify an image in which a lesion area appears and a kind of lesion by checking a transition of color on the lesion bar display unit 244.

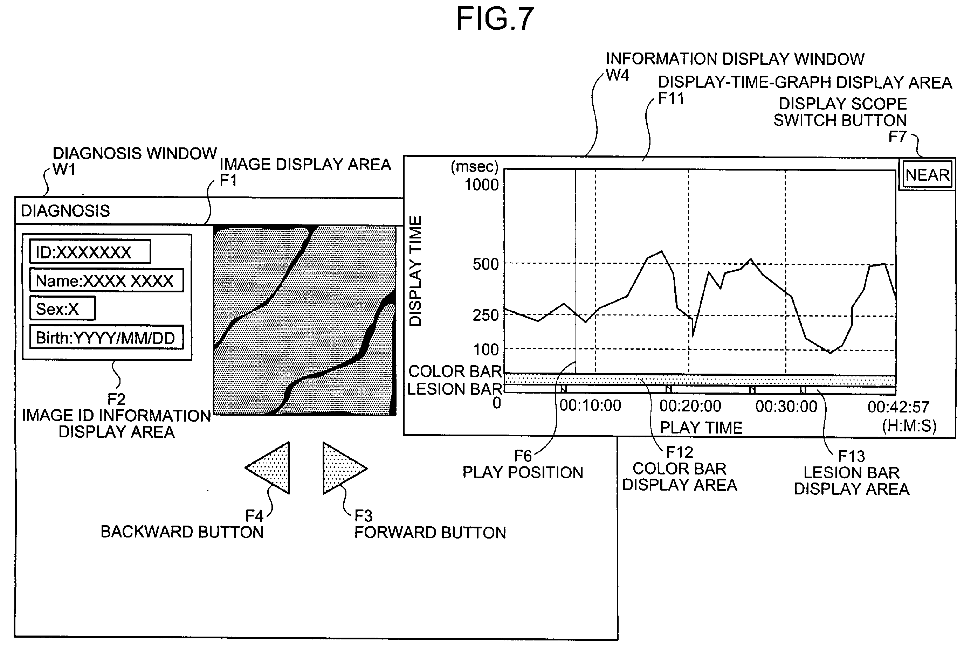

FIG. 7 is a view of a specific example of a display screen of the display unit 24. The image processing apparatus 6 displays, in addition to the diagnosis window W1, an information display window W4 in place of the information display window W2. In the information display window W4, a display-time-graph display area F11, a color bar display area F12, a lesion bar display area F13, and the display scope switch button F7 are displayed. In the display-time-graph display area F11, a display time graph similar to the display time graph shown on the display-time-graph display area F5 is displayed.

The color bar display area F12 represents a specific mode of the color bar display unit 243, and is a strip-shaped display area where an average color of an image corresponding to the play time is sequentially arranged as a bar parallel to a play time axis of the display time graph. The lesion bar display area F13 represents a specific mode of the lesion bar display unit 244, and is a strip-shaped display area where a color, attached by the lesion information calculating unit 213, of an image corresponding to the play time is sequentially arranged as a bar parallel to the play time axis of the display time graph.

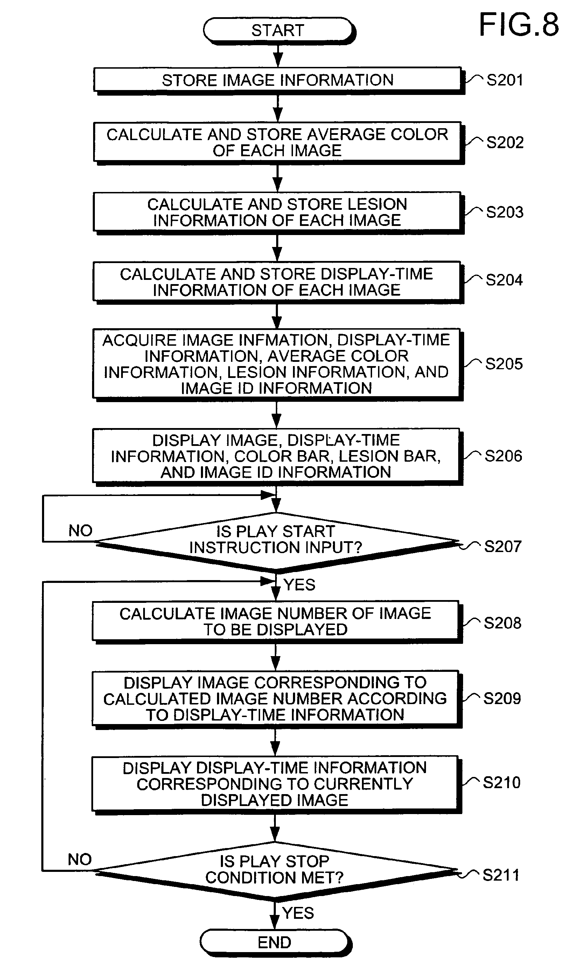

Next, a procedure to play the series of intracelomic images on the display unit 24, performed by the control unit 20 is described below with reference to FIG. 8. Firstly, the image processing unit 210 acquires the image information of the series of intracelomic images from the recording medium 4 via the card I/F 12, and stores the image information in the image information storage unit 131 (Step S201). The average color calculating unit 212 calculates the average color of each image, and stores the average color in the average color storage unit 233 (Step S202). Next, the lesion information calculating unit 213 calculates the lesion information of each image, and stores the lesion information in the lesion information storage unit 234 (Step S203). The display-time-information calculating unit 111 calculates the display-time information of each image, and stores the display-time information in the display-time-information storage unit 132 (Step S204). The display control unit 220 acquires information needed for the examiner to diagnose the subject 1, such as the image information, the display-time information, the average color information, the lesion information, and the image ID information (Step S205). Respective units of the display control unit 220 display the image, the display-time information, the color bar, the lesion bar, and the image ID information on the corresponding units of the display unit 24 (Step S206). At this point, the examiner can grasp, in addition to the total play time and the change in the display time, information on a transition of average color of image, information on an image which is determined to contain an image of a lesion area, and information on a kind of a lesion.

After that, the respective units of the display control unit 220 perform processes similar to the processes at Steps S105 to S109 performed by the units of the display control unit 120 (Steps S207 to S211). If the play start instruction is input (Step S207: Yes), the image display control unit 121 calculates the image number of the image to be displayed by the image display unit 141 (Step S208), and displays an image corresponding to the image number according to the display-time information (Step S209). The display-time-information display control unit 122 displays the display-time information corresponding to the image currently displayed on the image display unit 141 (Step S210). The display control unit 220 repeats the processes at Steps S207 to S210 until the play stop condition is met, and when the play stop condition is met (Step S211), the display control unit 220 stops playing the images.

Although not shown, when the display time graph of the near images is displayed on the information display window W4, the color bar display control unit 224 and the lesion bar display control unit 225 control the display unit 24 to display the color bar and the lesion bar corresponding to the scope of the play time displayed on the display time graph, respectively.

As described, with the image processing apparatus 6, the examiner can grasp, in addition to the display-time information of each image, information on the changes of color tone of each image and information on an image which is determined to contain an image of a lesion area, before each image is displayed, whereby the examiner can determine in advance which image he/she should pay attention to, and observe the image without fail. As a result, oversight of images decreases and the work burden caused by reviewing the images and the like can be lightened.

The image processing apparatus according to the second embodiment may display only one of the color bar and the lesion bar.

Third Embodiment

Now, a third embodiment of the present invention is described. In the first embodiment and the second embodiment, the display-time information and the like of each image are displayed when the series of intracelomic images are played, whereas, in the third embodiment, further, the display-time information may be changed.

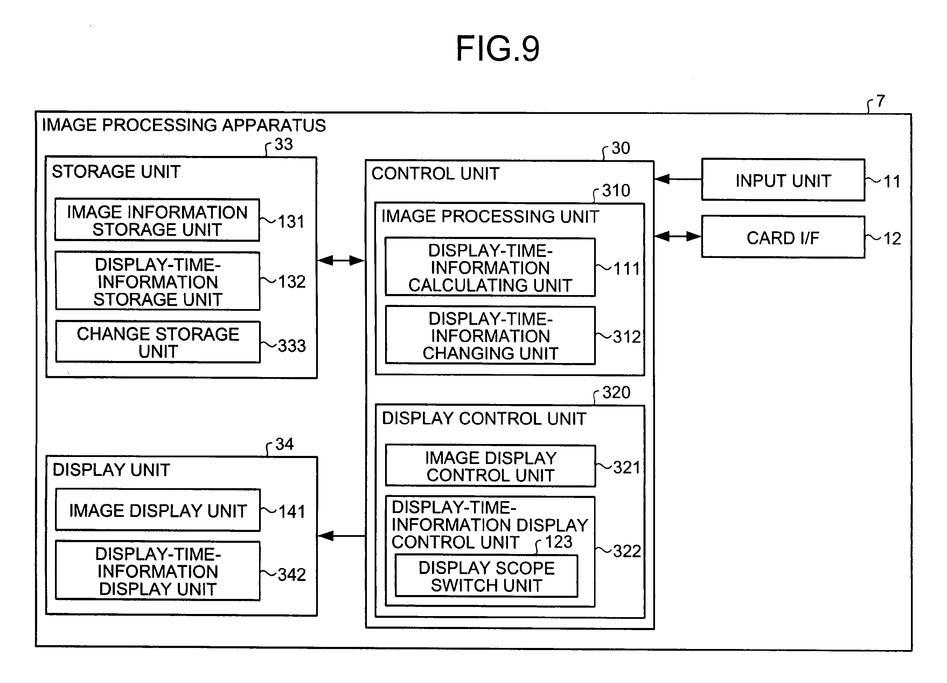

In the third embodiment, an image processing apparatus 7 is provided in place of the image processing apparatus 5 in the intra-subject information acquiring system of the first embodiment. FIG. 9 is a block diagram of a configuration of the image processing apparatus 7 according to the third embodiment. As shown in FIG. 9, the image processing apparatus 7 includes a control unit 30, a storage unit 33, and a display unit 34 in place of the control unit 10, the storage unit 13, and the display unit 14 included in the image processing apparatus 5. The control unit 30 includes an image processing unit 310 and a display control unit 320. The image processing unit 310 includes, in addition to the display-time-information calculating unit 111, a display-time-information changing unit 312, and the display control unit 320 includes an image display control unit 321 and a display-time-information display control unit 322. The display-time-information display control unit 322 includes the display scope switch unit 123 similarly to the display-time-information display control unit 122. The storage unit 33 includes, in addition to the units of the storage unit 13, a change storage unit 333. The display unit 34 includes, in addition to the image display unit 141, a display-time-information display unit 342. Other configurations are same as the configurations of the intra-subject information acquiring system and the image processing apparatus 5 according to the first embodiment, and a same numeral is attached to a same element.

The display-time-information changing unit 312 changes the display-time information based on information on a change of the display-time information acquired from the examiner through the input unit 11, and stores the changed display-time information in the change storage unit 333. When the display-time information is changed, the image display control unit 321 plays the series of intracelomic images based on the changed display-time information, and the display-time-information display control unit 322 controls the display unit 34 to display the changed display-time information.

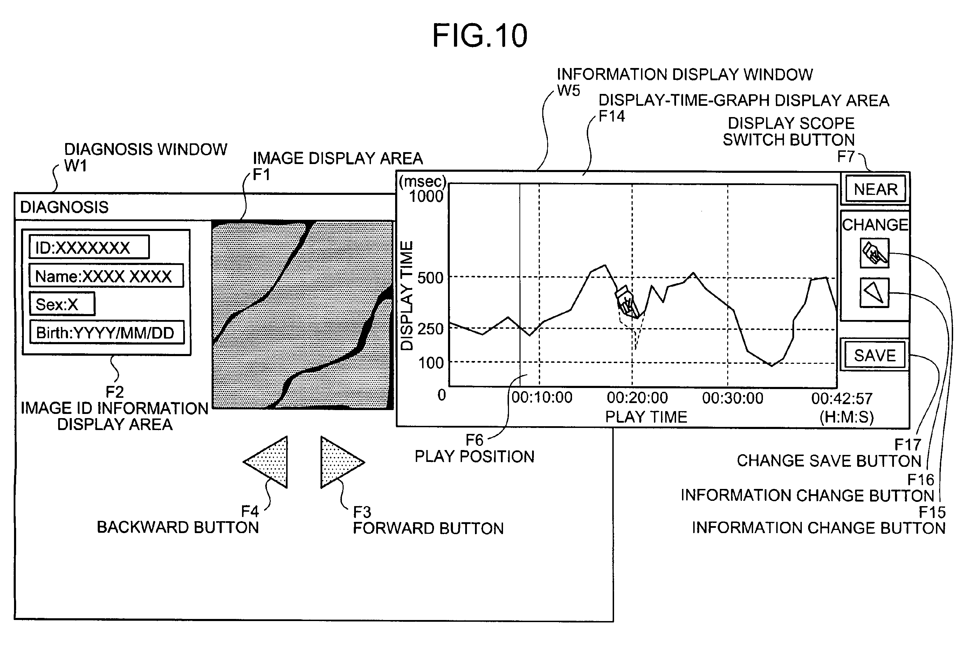

Information on the change of the display-time information is input by the examiner, for example, via a GUI screen. An example of the change of the display-time information through the GUI screen is described below with reference to FIG. 10. FIG. 10 is a view of a specific mode of a display screen of the display unit 32. The image processing apparatus 7 displays, in addition to the diagnosis window W1, an information display window W5 in place of the information display window W2. The information display window W5 displays a display-time-graph display area F14, information change buttons F15 and F16, a change save button F17, and the display scope switch button F7. Before the display-time information is changed, a display time graph similar to the display time graph displayed in the display-time-graph display area F5 is displayed in the display-time-graph display area F14.

When the examiner clicks the information change button F15 or F16 using a pointer on the display screen, a shape of the pointer changes into a same shape with an icon of the information change button F15 or F16. The examiner moves the pointer whose shape has changed to a position corresponding to an image whose display time is desired to be changed on the display time graph, clicks at the position, and drags the pointer along the display time axis. Here, as shown in FIG. 10, the display time graph is deformed as the pointer moves (solid line). The display time graph before the change is displayed in a dot line. The display time graph is fixed at the position where the examiner releases the pointer, and thus the display-time information is changed. Thus, the examiner inputs information on the change of the display-time information into the control unit 30.

When the information on the change of the display-time information is input after the information change button F15 is selected, the display-time-information changing unit 312 changes the display time of a predetermined number of images before and after an image corresponding to a clicked position on the display time graph in time series. On the other hand, when the information on the change of the display-time information is input after the information change button F16 is selected, the display-time-information changing unit 312 changes the display time of only the image corresponding to a clicked position on the display time graph.

For example, when the examiner inputs an instruction to change the display time of an image of an image number k by display time v after selecting the information change button F15, the display-time-information changing unit 312 changes the display time of images from an image of an image number k-m to an image of an image number k+m, using following formula (2). Here, m represents a predetermined number, and in the formula (2), j represents the image number, $T_j$ represents unmodified display time of an image of the image number j, and $T_j'$ represents modified display time of the image of the image number j.

$$[\text{Formula } 2]$$

$$T_j' = T_j + v \times \frac{m - |j - k|}{m} \qquad (2)$$

A manner of changing the display-time information of an image selected by the examiner and its near images is not limited to the manner using the formula (2). The display-time information may be changed, for example, by setting points corresponding to images of image numbers m-k, k, and m+k on the display time graph as control points and drawing a spline curve to change the display time graph.

When the examiner selects the change save button F17 after inputting the information on the change of the display-time information, the image processing unit 310 performs a process to store changed display-time information in the change storage unit 333. When the series of intracelomic images are played, the examiner may play the series of intracelomic images based on the display-time information before the change, on a display screen not shown. Alternatively, the image processing apparatus may be configured so that the examiner can select whether to play the series of intracelomic images based on the changed display-time information or not. Then, even when the same images are played again, the examiner can play the images based on the display-time information which is changed in advance.

With the image processing apparatus 7, since the examiner can change the display-time information of each image according to his/her needs, the examiner can observe the series of intracelomic images more conveniently. Further, the examiner can prevent the oversight of the images by changing the display time, whereby work burden caused from overlooking and reviewing the images and the like can be lightened.

Although the image processing apparatus 7 is described as including the display-time-information changing unit 312 and the change storage unit 333 in addition to the elements of the image processing apparatus 5, alternatively, the image processing apparatus 7 may be configured based on the configuration of the image processing apparatus 6. Then, similarly to the second embodiment, the display-time information, the color bar, and the lesion bar are displayed on the display screen, whereby the examiner can change the display time of each image properly based on the information displayed.

Further, the image processing apparatus according to the present invention can be employed for playing a series of images other than the intracelomic images picked up by the capsule endoscope 2.

INDUSTRIAL APPLICABILITY

As can be seen from the foregoing, the image processing apparatus, image processing program, and image processing method according to the present invention are useful for sequentially displaying images, in particular, intracelomic images picked up in a gastroenteric tract.

**Claims**

1. An image processing apparatus comprising:

   an image storage unit that stores a series of images;
   a display-time-information storage unit that stores display-time information which defines display time of each image;
   an image display unit that sequentially displays each image based on the display-time information;
   a display-time-information display unit that displays the display-time information at least of images to be displayed in time series after an image currently displayed on the image display unit; and
   a display control unit that performs a control to display an image on the image display unit, and to display the display-time information on the display-time-information display unit, based on the images stored in the image storage unit and the display-time information stored in the display-time-information storage unit.

2. The image processing apparatus according to claim 1, wherein
   the display control unit performs a control to display on the display-time-information display unit, a marker that indicates a position in time series of the image currently displayed on the image display unit.

3. The image processing apparatus according to claim 1 or 2, wherein
   the display control unit performs a control to display as the display-time information at least one of the display time which indicates time each image is displayed on the image display unit, a coefficient for determining the display time, a frame rate which indicates speed of display on the image display unit, and a value for determining relative display time of each image.

4. The image processing apparatus according to any one of claims 1 to 3, wherein
   the display control unit performs a control to display the display-time information of all images in the series of images which is a display target.

5. The image processing apparatus according to any one of claims 1 to 3, wherein
   the display control unit performs a control to display the display-time information of near images among the series of images which is a display target, the near images being a predetermined number of images displayed in time series before and after the image currently displayed on the image display unit, or images to be displayed in time series before and after the image currently displayed on the image display unit within a predetermined time period before and after the image currently displayed on the image display unit.

6. The image processing apparatus according to any one of claims 1 to 3, wherein
   the display control unit has a function of performing a control to display the display-time information of all images in the series of images whih is a display target, and of performing a control to display the display-time information of near images among the series of images which is the display target, the near images being a predetermined number of images displayed in time series before and after the image currently displayed on the image display unit, or images to be displayed in time series before and after the image currently displayed on the image display unit within a predetermined time period before and after the image currently displayed on the image display unit, and
   the display control unit further includes a display scope switch unit that switches the display-time information displayed on the display-time-information display unit between the display-time information of the all images and the display-time information of the near images, and performs a control to display one of the display-time information of the all images and the display-time information of the near images switched to by the display scope switch unit.

7. The image processing apparatus according to any one of claims 1 to 6, wherein
   the display control unit performs a control to display the display-time information in association with at least one of play time which is time spent on sequentially displaying the series of images, imaging time at which each image is taken, and an image number attached to each image in an order of imaging.

**8.** The image processing apparatus according to any one of claims 1 to 7, wherein
the image storage unit stores a series of intracelomic images taken inside a living body.

**9.** The image processing apparatus according to claim 8, wherein
the display control unit performs a control to display on the display-time-information display unit, at least one of information indicating a transition of color tone of the series of intracelomic images and information indicating whether an image of a lesion appears in each intracelomic image, corresponding to the display-time information.

**10.** The image processing apparatus according to any one of claims 1 to 9, further comprising
a display-time-information changing unit that acquires information to change the display-time information, and changes the display-time information, and
the display control unit performs a control to display each image based on the display-time information changed by the display-time-information changing unit.

**11.** The image processing apparatus according to claim 10, further comprising
a change storage unit that stores the display-time information changed by the display time changing unit.

**12.** An image processing program causing a computer to perform:

a storing process to store display-time information which defines display time of each image stored in an image storage unit that stores a series of images; and
a display control process to perform a control to sequentially display each image stored in the image storage unit on an image display unit according to the display-time information, and to display the display-time information at least of images to be displayed in time series after an image currently displayed on the image display unit based on the display-time information stored in the display-time-information storage unit.

**13.** An image processing method comprising:

a storing step of storing display-time information which defines display time of each image stored in an image storage unit that stores a series of images; and
a display control step of performing a control to sequentially display each image stored in the image storage unit on an image display unit according to the display-time information, and to display the display-time information at least of images to be displayed in time series after an image currently displayed on the image display unit based on the display-time information stored in the display-time-information storage unit.

# FIG.1

CAPSULE ENDOSCOPE
2

1 SUBJECT

IMAGE PROCESSING
APPARATUS
5

RECEIVING
APPARATUS
3

4
RECORDING MEDIUM

# FIG.2

IMAGE PROCESSING APPARATUS ⌐5

STORAGE UNIT ⌐13
- IMAGE INFORMATION STORAGE UNIT ～131
- DISPLAY-TIME-INFORMATION STORAGE UNIT ～132

CONTROL UNIT ⌐10

IMAGE PROCESSING UNIT ⌐110
- DISPLAY-TIME-INFORMATION CALCULATING UNIT ～111

DISPLAY CONTROL UNIT ⌐120
- IMAGE DISPLAY CONTROL UNIT ～121
- DISPLAY-TIME-INFORMATION DISPLAY CONTROL UNIT ～122
  - DISPLAY SCOPE SWITCH UNIT ⌐123

INPUT UNIT ～11

CARD I/F ～12

DISPLAY UNIT ⌐14
- IMAGE DISPLAY UNIT ～141
- DISPLAY-TIME-INFORMATION DISPLAY UNIT ～142

EP 2 174 580 A1

# FIG.3

INFORMATION DISPLAY WINDOW
W2
DISPLAY-TIME-GRAPH DISPLAY AREA
F5
DISPLAY SCOPE
SWITCH BUTTON
F7

IMAGE DISPLAY AREA
F1

W1
DIAGNOSIS WINDOW

DIAGNOSIS

ID:XXXXXXX

Name:XXXX XXXX

Sex:X

Birth:YYYY/MM/DD

F2
IMAGE ID INFORMATION
DISPLAY AREA

(msec)
1000

DISPLAY TIME

500

250

100

0    00:10:00    00:20:00    00:30:00    00:42:57
PLAY TIME                    (H:M:S)

NEAR

F6
PLAY POSITION

F4
BACKWARD BUTTON

F3
FORWARD BUTTON

EP 2 174 580 A1

# FIG.4

START

STORE IMAGE INFORMATION — S101

CALCULATE AND STORE DISPLAY-TIME INFORMATION OF EACH IMAGE — S102

ACQUIRE IMAGE INFORMATION, DISPLAY-TIME INFORMATION, AND IMAGE ID INFORMATION — S103

DISPLAY IMAGE, DISPLAY-TIME INFORMATION, AND IMAGE ID INFORMATION — S104

IS PLAY START INSTRUCTION INPUT? — S105

NO

YES

CALCULATE IMAGE NUMBER OF IMAGE TO BE DISPLAYED — S106

DISPLAY IMAGE CORRESPONDING TO CALCULATED IMAGE NUMBER ACCORDING TO DISPLAY-TIME INFORMATION — S107

DISPLAY DISPLAY-TIME INFORMATION CORRESPONDING TO CURRENTLY DISPLAYED IMAGE — S108

IS PLAY STOP CONDITION MET? — S109

NO

YES

END

# FIG.5

INFORMATION DISPLAY WINDOW
W3

DISPLAY-TIME-GRAPH DISPLAY AREA
F8

DISPLAY SCOPE
SWITCH BUTTON
F10

DIAGNOSIS WINDOW
W1

IMAGE DISPLAY AREA
F1

DIAGNOSIS

ID:XXXXXXX

Name:XXXX XXXX

Sex:X

Birth:YYYY/MM/DD

F2
IMAGE ID INFORMATION
DISPLAY AREA

ALL

(msec)
1000

DISPLAY TIME

500

250

100

0

00:07:16   00:08:16        00:09:16
PLAY TIME        (H:M:S)

F9
PLAY POSITION

F4
BACKWARD BUTTON

F3
FORWARD BUTTON

EP 2 174 580 A1

# FIG.6

**IMAGE PROCESSING APPARATUS** ～6

**STORAGE UNIT** ～23
- IMAGE INFORMATION STORAGE UNIT ～131
- DISPLAY-TIME-INFORMATION STORAGE UNIT ～132
- AVERAGE COLOR STORAGE UNIT ～233
- LESION INFORMATION STORAGE UNIT ～234

**CONTROL UNIT** ～20

INPUT UNIT ～11

CARD I/F ～12

**IMAGE PROCESSING UNIT** ～210
- DISPLAY-TIME-INFORMATION CALCULATING UNIT ～111
- AVERAGE COLOR CALCULATING UNIT ～212
- LESION INFORMATION CALCULATING UNIT ～213

**DISPLAY CONTROL UNIT** ～220
- IMAGE DISPLAY CONTROL UNIT ～121
- DISPLAY-TIME-INFORMATION DISPLAY CONTROL UNIT ～122
  - DISPLAY SCOPE SWITCH UNIT ～123
- COLOR BAR DISPLAY CONTROL UNIT ～224
- LESION BAR DISPLAY CONTROL UNIT ～225

**DISPLAY UNIT** ～24
- IMAGE DISPLAY UNIT ～141
- DISPLAY-TIME-INFORMATION DISPLAY UNIT ～142
- COLOR BAR DISPLAY UNIT ～243
- LESION BAR DISPLAY UNIT ～244

# FIG.7

DIAGNOSIS WINDOW W1 — IMAGE DISPLAY AREA F1

DIAGNOSIS

ID:XXXXXXX

Name:XXXX XXXX

Sex:X

Birth:YYYY/MM/DD

F2
IMAGE ID INFORMATION
DISPLAY AREA

F4 — BACKWARD BUTTON

F3 — FORWARD BUTTON

INFORMATION DISPLAY WINDOW W4

DISPLAY-TIME-GRAPH DISPLAY AREA F11

DISPLAY SCOPE SWITCH BUTTON F7

NEAR

(msec)
1000

DISPLAY TIME

500

250

100

COLOR BAR
LESION BAR

0    00:10:00    00:20:00    00:30:00    00:42:57

PLAY TIME    (H:M:S)

F6 — PLAY POSITION

F12 — COLOR BAR DISPLAY AREA

F13 — LESION BAR DISPLAY AREA

EP 2 174 580 A1

FIG.8

START

STORE IMAGE INFORMATION — S201

CALCULATE AND STORE AVERAGE COLOR OF EACH IMAGE — S202

CALCULATE AND STORE LESION INFORMATION OF EACH IMAGE — S203

CALCULATE AND STORE DISPLAY-TIME INFORMATION OF EACH IMAGE — S204

ACQUIRE IMAGE INFMATION, DISPLAY-TIME INFORMATION, AVERAGE COLOR INFORMATION, LESION INFORMATION, AND IMAGE ID INFORMATION — S205

DISPLAY IMAGE, DISPLAY-TIME INFORMATION, COLOR BAR, LESION BAR, AND IMAGE ID INFORMATION — S206

IS PLAY START INSTRUCTION INPUT? — S207
NO
YES

CALCULATE IMAGE NUMBER OF IMAGE TO BE DISPLAYED — S208

DISPLAY IMAGE CORRESPONDING TO CALCULATED IMAGE NUMBER ACCORDING TO DISPLAY-TIME INFORMATION — S209

DISPLAY DISPLAY-TIME INFORMATION CORRESPONDING TO CURRENTLY DISPLAYED IMAGE — S210

IS PLAY STOP CONDITION MET? — S211
NO
YES

END

# FIG.9

IMAGE PROCESSING APPARATUS                                                    7

STORAGE UNIT                    33

- IMAGE INFORMATION STORAGE UNIT  131
- DISPLAY-TIME-INFORMATION STORAGE UNIT  132
- CHANGE STORAGE UNIT  333

CONTROL UNIT                    30

IMAGE PROCESSING UNIT           310

- DISPLAY-TIME-INFORMATION CALCULATING UNIT  111
- DISPLAY-TIME-INFORMATION CHANGING UNIT  312

DISPLAY CONTROL UNIT            320

- IMAGE DISPLAY CONTROL UNIT  321
- DISPLAY-TIME-INFORMATION DISPLAY CONTROL UNIT  123  322
  - DISPLAY SCOPE SWITCH UNIT  123

INPUT UNIT  11

CARD I/F  12

DISPLAY UNIT                    34

- IMAGE DISPLAY UNIT  141
- DISPLAY-TIME-INFORMATION DISPLAY UNIT  342

EP 2 174 580 A1

# FIG.10

INFORMATION DISPLAY WINDOW
W5

DISPLAY-TIME-GRAPH DISPLAY AREA
F14

DISPLAY SCOPE
SWITCH BUTTON
F7

DIAGNOSIS WINDOW
W1

IMAGE DISPLAY AREA
F1

DIAGNOSIS

ID:XXXXXXX

Name:XXXX XXXX

Sex:X

Birth:YYYY/MM/DD

F2
IMAGE ID INFORMATION
DISPLAY AREA

NEAR

CHANGE

SAVE

F17
CHANGE SAVE BUTTON

F16
INFORMATION CHANGE BUTTON

F15
INFORMATION CHANGE BUTTON

(msec)
1000

DISPLAY TIME

500

250

100

0

00:10:00    00:20:00    00:30:00    00:42:57

PLAY TIME                    (H:M:S)

F6
PLAY POSITION

F4
BACKWARD BUTTON

F3
FORWARD BUTTON

EP 2 174 580 A1

## INTERNATIONAL SEARCH REPORT

International application No.

PCT/JP2008/060300

A. CLASSIFICATION OF SUBJECT MATTER
*A61B1/00*(2006.01)i, *A61B5/07*(2006.01)i

According to International Patent Classification (IPC) or to both national classification and IPC

B. FIELDS SEARCHED

Minimum documentation searched (classification system followed by classification symbols)
A61B1/00, A61B5/07

Documentation searched other than minimum documentation to the extent that such documents are included in the fields searched

| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2006-55430 A (Olympus Corp.), | 1-4,8,10-13 |
| Y | 02 March, 2006 (02.03.06), | 5-7,9 |
| | Full text; all drawings | |
| | (Family: none) | |
| Y | JP 2006-280792 A (Olympus Corp.), | 5,6 |
| | 19 October, 2006 (19.10.06), | |
| | Full text; all drawings | |
| | (Family: none) | |
| Y | JP 2006-288612 A (Olympus Corp.), | 7,9 |
| | 26 October, 2006 (26.10.06), | |
| | Full text; all drawings | |
| | (Family: none) | |

☐ Further documents are listed in the continuation of Box C.   ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 12 August, 2008 (12.08.08) | 26 August, 2008 (26.08.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**EP 2 174 580 A1**

**REFERENCES CITED IN THE DESCRIPTION**

**Patent documents cited in the description**

- JP 2006280792 A

- JP 2005192880 A